(19) Europäisches Patentamt
European Patent Office
Office européen des brevets

(11) **EP 3 213 686 A1**

(12) **EUROPEAN PATENT APPLICATION**

(43) Date of publication:
06.09.2017 Bulletin 2017/36

(51) Int Cl.:
*A61B 5/1455* (2006.01)  *A61B 5/083* (2006.01)
*A61B 5/029* (2006.01)  *A61B 5/00* (2006.01)
*A61B 5/08* (2006.01)  *A61B 5/091* (2006.01)

(21) Application number: 17151757.6

(22) Date of filing: 17.01.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 04.03.2016 JP 2016041835

(71) Applicant: Nihon Kohden Corporation
Tokyo 161-8560 (JP)

(72) Inventors:
• Saeki, Kota
Tokyo 161-8560 (JP)
• Kobayashi, Naoki
Tokyo 161-8560 (JP)

(74) Representative: Hoffmann Eitle
Patent- und Rechtsanwälte PartmbB
Arabellastraße 30
81925 München (DE)

(54) **BIOLOGICAL INFORMATION MEASUREMENT DEVICE, MIXED VENOUS OXYGEN SATURATION ESTIMATION METHOD, AND PROGRAM**

(57)  Provided is a biological information measurement device (1), including: a blood index calculation section (12) which calculates at least part of a blood index associated with oxygen transport based on transmitted light or reflected light when an arbitrary body part of a test subject is irradiated with light with a plurality of wavelengths; an oxygen consumption calculation section (14) which calculates an oxygen consumption based on a gas concentration in inspired air, a gas concentration in expired air, and a ventilation amount of the test subject; a cardiac output calculation section (13) which calculates a cardiac output based on a biological signal acquired by a noninvasive method from the test subject; and an SvO2 estimation section (15) which estimates the mixed venous oxygen saturation of the test subject by substituting the blood index, the oxygen consumption, and the cardiac output in a calculation formula.

*FIG. 2*

| HEART RATE | |
|---|---|
| 88 | |
| BLOOD PRESSURE | |
| 120 / 70 | |
| SvO2 | |
| 75 | |
| SpO2 | |
| 99 | |
| BODY TEMPERATURE | |
| 36.5 | |
| RESPIRATORY RATE | |
| 19 | |

EP 3 213 686 A1

**Description**

Field of the Invention

The present invention relates to a biological information measurement device, a mixed venous oxygen saturation estimation method, and a program.

Background of the Invention

Mixed venous oxygen saturation (SvO2) is oxygen saturation of pulmonary arterial blood. The mixed venous oxygen saturation is used as an index of the balance between supply and demand of oxygen in the body. For example, a decrease in mixed venous oxygen saturation means a lack of oxygen supply or an increase in oxygen consumption. The mixed venous oxygen saturation changes due to respiratory failure, circulatory failure, or hypermetabolism accompanying fever or infection. Therefore, the mixed venous oxygen saturation is particularly utilized for ascertaining the state of circulation of oxygen in the whole body in the intensive care field.

In general, the mixed venous oxygen saturation is measured by inserting a Swan-Ganz catheter into the pulmonary artery. However, this method is an invasive method in which a Swan-Ganz catheter is inserted into the body of a test subject, and therefore places a large burden on the test subject.

In light of this, a method of noninvasively measuring the mixed venous oxygen saturation has been proposed. JP-T-2010-524598 (Patent Document 1) discloses a technique in which a light-emitting element and a light-receiving element are placed in the vicinity of a target deep vascular structure (for example, the pulmonary artery), and blood flowing through the deep vascular structure is irradiated with light, whereby the oxygen saturation of the blood is measured.

WO 13/112812 (Patent Document 2) discloses a system in which the mixed venous oxygen saturation is noninvasively calculated using photoacoustic imaging. In the system, a tissue to be measured is irradiated with laser light, and the mixed venous oxygen saturation is calculated using an ultrasonic wave generated accompanying light absorption.

U.S. Patent Application Publication No 2012-0065485 (Patent Document 3) discloses a device in which pulmonary arterial blood is irradiated with light using an NIRS sensor and the mixed venous oxygen saturation is measured.

In the techniques disclosed in the above Patent Documents 1 and 2, the mixed venous oxygen saturation is noninvasively measured by applying light (including laser light) from a biological tissue immediately above the pulmonary artery. In this method, in the case where light could not be applied to the exact place (the place where a blood vessel to be measured is present), the accurate mixed venous oxygen saturation may not be able to be measured. Further, in the technique disclosed in Patent Document 3, since an NIRS sensor is used, light does not reach the deep region in the body, and thus, the measurement may not be able to be performed accurately in a test subject with a large physique.

That is, in the above-mentioned techniques, the mixed venous oxygen saturation may not be able to be measured accurately and noninvasively.

Summary of the Invention

The invention has been made in view of the above problems, and a main object of the invention is to provide a biological information measurement device and a mixed venous oxygen saturation estimation method capable of noninvasively and accurately measuring the mixed venous oxygen saturation.

One aspect of a biological information measurement device according to the invention includes:

a blood index calculation section which calculates at least part of a blood index associated with oxygen transport based on transmitted light or reflected light when an arbitrary body part of a test subject is irradiated with light with a plurality of wavelengths;
an oxygen consumption calculation section which calculates an oxygen consumption based on a gas concentration in inspired air, a gas concentration in expired air, and a ventilation amount of the test subject;
a cardiac output calculation section which calculates a cardiac output based on a biological signal acquired by a noninvasive method from the test subject; and
an Sv02 estimation section which estimates the mixed venous oxygen saturation of the test subject by substituting the blood index, the oxygen consumption, and the cardiac output in a calculation formula derived from the law of blood circulation and an oxygen binding amount per unit hemoglobin.

It is known that a blood index (an arterial oxygen saturation or the amount of hemoglobin in blood) associated with oxygen transport, an oxygen consumption, and a cardiac output can be noninvasively and accurately acquired by the function of a biological information monitor used in a general hospital (JP-A-8-322822 (Patent Document 4), JP-A-2005-312947 (Patent Document 5), etc.). An Sv02 estimation section 15 estimates a mixed venous oxygen saturation (Sv02) by substituting these parameters in a calculation formula derived from the blood circulation and the oxygen transport amount per unit hemoglobin. The Sv02 estimation section 15 uses parameters which can be noninvasively and accurately calculated in the calculation, and therefore, the mixed venous oxygen saturation (Sv02) can be continuously and accurately estimated.

The invention can provide a biological information measurement device and a mixed venous oxygen saturation estimation method capable of noninvasively and accurately measuring the mixed venous oxygen saturation.

Brief Description of the Drawings

Fig. 1 is a block diagram showing the configuration of a biological information measurement device 1 according to a first embodiment.
Fig. 2 is a view showing an example of a display screen of the biological information measurement device 1 according to the first embodiment.

Detailed Description of the Invention

First Embodiment

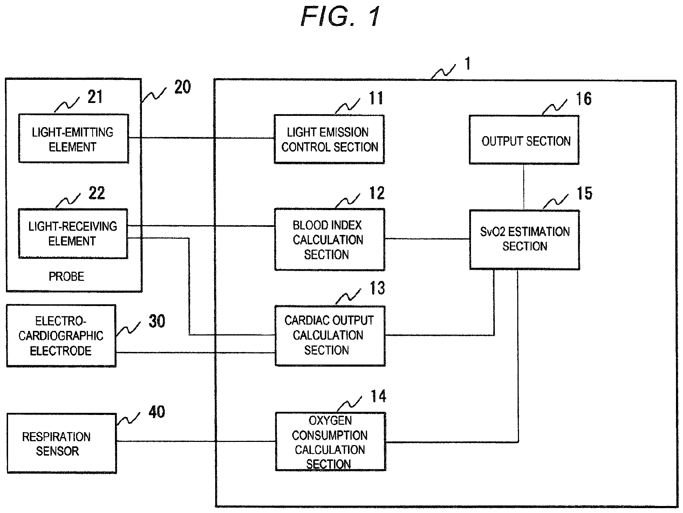

Hereinafter, embodiments of the invention will be described with reference to the drawings. Fig. 1 is a block diagram showing the configuration of a biological information measurement device 1 according to a first embodiment. The biological information measurement device 1 measures various vital signs (electrocardiogram, blood pressure, arterial oxygen saturation, heart rate, respiratory rate, body temperature, etc.) of a test subject. The biological information measurement device 1 may be a bed-side monitor which is used in a hospital room, or may be a medical telemeter (transmitter) which is always carried by a test subject (patient). That is, the biological information measurement device 1 may have any form or size as long as it is a device which measures the vital signs of a test subject.

The biological information measurement device 1 is connected to any type of probe, a cuff, an electrode, and the like. The biological information measurement device 1 shown in Fig. 1 is connected to a probe 20, an electrocardiographic electrode 30, and a respiration sensor 40. Incidentally, although not shown in the drawing, the biological information measurement device 1 may be connected to a cuff (air bag) for noninvasive blood pressure measurement, a body temperature sensor, or the like.

The probe 20 emits light on an arbitrary body part (for example, a fingertip, an earlobe, a forehead, or the like) of a test subject, and receives transmitted light or reflected light from the body part. The probe 20 includes a light-emitting element 21 and a light-receiving element 22. The light-emitting element 21 emits light with a plurality of wavelengths including a wavelength of light which is absorbed by water on a body part. The light-emitting element 21 emits light with a plurality of wavelengths selected from an arbitrary wavelength between, for example, 600 nm and 1300 nm on a biological tissue. The light-emitting element 21 may be any as long as it is constituted by, for example, a light-emitting diode (LED).

The light-receiving element 22 receives transmitted light obtained by transmitting the light with a plurality of wavelengths through the body part (or reflected light obtained by reflecting the light with a plurality of wavelengths from the body part). The light-receiving element 22 converts the transmitted light (or the reflected light) into an electrical signal (biological signal) and inputs the signal to a blood index calculation section 12 and a cardiac output calculation section 13. Although not shown in the drawing, the light-receiving element 22 may include an A/D (analog-digital) converter which performs A/D conversion of the electrical signal as appropriate. The light-receiving element 22 may be any as long as it is constituted by, for example, a photodiode.

The electrocardiographic electrode 30 includes a plurality of electrodes (a disposable electrode and a clip electrode) for measuring an electrocardiogram (ECG) of a test subject. The electrocardiographic electrode 30 is attached to a predetermined place of the chest or the limb of a test subject. The electrocardiographic electrode 30 and the biological information measurement device 1 are connected to each other through an electrical cable. The electrocardiographic electrode 30 inputs a biological signal acquired from the predetermined place of the test subject to the cardiac output calculation section 13.

The respiration sensor 40 measures a gas concentration in expired air, a gas concentration in inspired air, and a ventilation amount of a test subject. The respiration sensor 40 may be actually constituted by a plurality of sensor devices. For example, the respiration sensor 40 may be constituted by an artificial respirator which can be connected to the so-called biological information measurement device 1 and a gas sensor and a flow sensor which can be attached to the artificial respirator. A medical worker places the gas sensor and the flow sensor on an expired air-side respiratory circuit and an inspired air-side respiratory circuit or an exhaust port of the artificial respirator. The gas sensor acquires the expired air and inspired air of the test subject from the respective respiratory circuits or the exhaust port and measures a gas concentration in the expired air and a gas concentration in the inspired air. Further, the flow sensor measures a ventilation amount of the test subject.

Further, the respiration sensor 40 may measure a gas concentration in the expired air or a gas concentration in the inspired air by a so-called side-stream system. The respiration sensor 40 is constituted by, for example, an artificial nose attached to a test subject, an adapter for sampling, a sampling tube, a gas sensor, a flow sensor to be attached to the mouth, and the like. The expired air of a test subject is sucked through the sampling tube, and the sucked gas is measured by the gas sensor. By doing this, a gas concentration in the expired air and a gas concentration in the inspired air of the test subject are measured. The flow sensor attached to the mouth measures a ventilation amount of the test subject.

Further, the respiration sensor 40 may be constituted by a gas sensor and a flow sensor attached to the mouth. The gas sensor in this configuration measures a gas concentration in expired air and a gas concentration in inspired air of a test subject. The flow sensor measures a ventilation amount of the test subject.

That is, the respiration sensor 40 may have any configuration as long as it can measure a gas concentration in expired air, a gas concentration in inspired air, and a ventilation amount of a test subject. The respiration sensor 40 inputs the measured gas concentration in the expired air, gas concentration in the inspired air, and ventilation amount of the test subject to an oxygen consumption calculation section 14.

Subsequently, the configuration of the biological information measurement device 1 will be described. The biological information measurement device 1 includes a light emission control section 11, a blood index calculation section 12, a cardiac output calculation section 13, an oxygen consumption calculation section 14, an Sv02 estimation section 15 , and an output section 16. Although not shown in the drawing, the biological information measurement device 1 includes a memory section (including a primary memory device and a secondary memory device) which stores any type of data.

The light emission control section 11 controls light with a plurality of wavelengths emitted on a body part of a test subject. Specifically, the light emission control section 11 controls the wavelength, emission timing, and emission intensity of light emitted by the light-emitting element 21. Here, it is preferred that the light emission control section 11 controls emission of light so that light with a plurality of wavelengths including light with a wavelength which is absorbed by water is emitted in order for the below-mentioned blood index calculation section 12 to calculate the amount of hemoglobin in blood.

The blood index calculation section 12 calculates at least part of a blood index associated with oxygen transport based on light (transmitted light or reflected light) obtained by transmitting or reflecting the light with a plurality of wavelengths including a wavelength of light which is absorbed by water through or from an arbitrary body part of a test subject. More specifically, the blood index calculation section 12 calculates at least an arterial oxygen saturation, and preferably also calculates the amount of hemoglobin in blood.

The blood index calculation section 12 acquires transmitted light or reflected light when red light (for example, light with a wavelength of 660 nm) and infrared light (for example, light with a wavelength of 940 nm) are emitted on a body part. The absorbance of hemoglobin in blood for red light and infrared light varies whether or not the hemoglobin binds to oxygen. The recognition of an artery may be performed by known plethysmography. The blood index calculation section 12 calculates the arterial oxygen saturation from the ratio of absorbed red light to absorbed infrared light (pulse spectrophotometry). Incidentally, a detailed calculation method of the arterial oxygen saturation may be basically the same as the method described in Non-Patent Document 1 ("Pulse Oximeter no Tanjo to Riron (Advent and Theory of Pulse Oximeter)", written by Takuo Aoyagi, The Journal of Japan Society for Clinical Anesthesia, vol. 10, No. 1, pp. 1-11, 1990) or Non-Patent Document 2 ("ME Hayawakari (Quick Understanding) Q&A, Sphygmomanometer, Cardiac Output Meter, Blood Flow Meter" supervised by Yasuhisa Sakurai, Nankodo Co., Ltd.).

Further, the blood index calculation section 12 calculates the amount of hemoglobin in blood using pulse spectrophotometry. The pulse spectrophotometry is a method of determining the ratios of the concentrations of substances in arterial blood by irradiating a biological tissue with light and measuring the light absorption property of arterial blood in the tissue utilizing the fact that the effective thickness of blood is pulsated by blood pulsation in the biological tissue. Various types of hemoglobin in blood (for example, oxygenated hemoglobin, deoxygenated hemoglobin, carboxyhemoglobin, and methemoglobin) and water have different light absorption properties, and therefore, by using light with a plurality of wavelengths, the ratios of the concentrations of various types of hemoglobin and water can be obtained. By calculating the ratio ($CHb/Cw = Hb$ (g/ml)) of the sum of the ratios of the concentrations of various types of hemoglobin ($CHb$ (g%)) with respect to the ratio of the concentration of water ($Cw$ (g%)), the concentration of hemoglobin can be calculated. Incidentally, in this calculation, the volume of 1 g of water is assumed to be 1 ml. Therefore, in the case where the amount of hemoglobin in blood is calculated, it is necessary to irradiate a body part with light with a wavelength which is absorbed by water. Please see Patent Document 4 for a detailed calculation method of the amount of hemoglobin in blood using pulse spectrophotometry.

It is more preferred that the blood index calculation section 12 calculates the amount of hemoglobin in blood, in which only hemoglobin capable of transferring oxygen (normal hemoglobin) is targeted (in other words, abnormal hemoglobin is excluded). That is, it is desired that the fractional oxygen saturation which is the ratio of oxygenated hemoglobin capable of transferring oxygen to the total hemoglobin is used. Alternatively, the concentration of oxygenated hemoglobin Hb02 (g/dl) may be directly calculated by calculating the ratio of the concentration of water Cw (g%) with respect to the ratio of the concentration of the above-mentioned oxygenated hemoglobin CHb02 (g%).

The abnormal hemoglobin refers to hemoglobin which does not have an ability to transfer oxygen, and is, for example, carboxyhemoglobin or methemoglobin.

In the case where the concentration of carboxyhemoglobin or the concentration of methemoglobin is calculated, it is only necessary to allow the light-emitting element 21 to irradiate a test subject with orange light (or red-orange light) in addition to near infrared light and red light as light to be irradiated onto the test subject. The transmitted light (or the reflected light) of each light changes in response to blood pulsation. The blood index calculation section 12 determines an extinction ratio between respective wavelengths, and the concentration of carboxyhemoglobin or the concentration of methemoglobin may be calculated based on the extinction ratio. Please see JP-A-2002-228579 (Patent Document 6) which is the earlier application filed by the present inventors for a detailed calculation method of the concentration of carboxyhemoglobin. Similarly, please see JP-A-2002-315739 (Patent Document 7) which is the earlier application filed by the present inventors for a detailed calculation method of the concentration of methemoglobin. Since the concentration of abnormal hemoglobin can be calculated, the amount of normal hemoglobin can be calculated by multiplying the total amount of hemoglobin by the concentration of normal hemoglobin.

The blood index calculation section 12 inputs the calculated arterial oxygen saturation and the calculated hemoglobin amount in blood to the SvO2 estimation section 15. As described above, the blood index calculation section 12 may input the total amount of hemoglobin in blood to the Sv02 estimation section 15 as the amount of hemoglobin in blood, however, it is more preferred that a value (the amount of normal hemoglobin) obtained by subtracting the amount of abnormal hemoglobin from the total amount of hemoglobin in blood is input to the Sv02 estimation section 15 as the amount of hemoglobin in blood.

The cardiac output calculation section 13 calculates a cardiac output based on the biological signal acquired from a test subject. This will be described in detail below.

The cardiac output calculation section 13 acquires an electrocardiographic signal from the electrocardiographic electrode 30 and calculates an electrocardiogram (ECG) from the electrocardiographic signal. The cardiac output calculation section 13 calculates a heart rate (HR) based on the waveform of the electrocardiogram. For example, the cardiac output calculation section 13 may calculate a heart rate from the number of detected R waves.

Further, the cardiac output calculation section 13 acquires a photoplethysmographic signal from the probe 20. As described above, the probe 20 performs light emission for measuring a general arterial oxygen saturation (Sa02). The cardiac output calculation section 13 calculates a pulse wave transition time (PWTT) based on a photoplethysmographic waveform acquired from the photoplethysmographic signal and the electrocardiogram. Please see, for example, Non-Patent Document 3 (Internet <URL: http://www.nihonkohden.co.jp/iryo/techinfo/pwtt/principle. html> searched on February 13, 2016) for the relationship among the photoplethysmographic waveform, the electrocardiogram, and the pulse wave transition time. Further, as for the detailed calculation method of the pulse wave transition time, the calculation may be performed by utilizing a pulse pressure, and for example, the same method as described in Patent Document 5 may be adopted.

The cardiac output calculation section 13 estimates a cardiac output by substituting the pulse wave transition time and the heart rate in the following formula (1).

$$\mathrm{CO = (\alpha L \times PWTT + \beta L) \times HR} \qquad \mathrm{Formula\ (1)}$$

In the formula (1), CO represents a cardiac output, PWTT represents a pulse wave transition time, HR represents a heart rate, and $\alpha$, $\beta$, and L each represent a coefficient intrinsic to a test subject.

The respective coefficients in the above formula (1) are coefficients intrinsic to a test subject, however, these coefficients may be calibrated using calibration values obtained by the measurement of a blood pressure for calibration. As for a detailed calibration method, a method equivalent to the method described in Patent Document 5 (for example, Fig. 12 in Patent Document 5) may be adopted.

The cardiac output calculation section 13 inputs the calculated cardiac output to the Sv02 estimation section 15. When using the above formula (1), the cardiac output can be continuously calculated. Therefore, it is preferred that the cardiac output calculation section 13 continuously calculates the cardiac output and inputs the calculated cardiac output to the Sv02 estimation section 15.

To the oxygen consumption calculation section 14, a gas concentration in the expired air, a gas concentration in the inspired air, and a ventilation amount of a test subject are input from the respiration sensor 40. The oxygen consumption calculation section 14 calculates a difference between the oxygen concentration in the gas concentration in the expired air and the oxygen concentration in the gas concentration in the inspired air (difference in oxygen concentration). The oxygen consumption calculation section 14 calculates the oxygen consumption (V02) of the test subject by multiplying the calculated difference in oxygen concentration by the ventilation amount. The oxygen consumption calculation section 14 inputs the calculated oxygen consumption to the Sv02 estimation section 15.

The SVO2 estimation section 15 estimates the mixed venous oxygen saturation based on the amount of hemoglobin in blood, the arterial oxygen saturation, the oxygen consumption, and the cardiac output. The mechanism of this estimation will be described below.

It has been widely known that the Fick's formula (the following formula (2)) based on the law of blood circulation (the law of conservation of mass) is established (Non-Patent Document 4 (Yung GL, et.al, "Comparison of impedance cardiography to direct Fick and thermodilution cardiac output determination in pulmonary arterial hypertension.", Congestive Heart Failure 2004, 10 (2, suppl. 2): 7-10), Internet <URL: http://www.ncbi.nlm.nih.gov/pubmed/15073478> searched on February 13, 2016)).

$$(CaO2-CvO2) \times CO = VO2 \qquad \text{Formula (2)}$$

In the formula (2), Ca02 represents an arterial oxygen content, Cv02 represents a mixed venous oxygen content, CO represents a cardiac output, and V02 represents an oxygen consumption.

Further, it is known that the amount of oxygen binding to 1 g of hemoglobin is from about 1.3 ml to 1.4 ml (Non-Patent Document 4) . When this oxygen binding amount per gram of hemoglobin is represented by K (K is a constant between 1.3 and 1.4, preferably between 1.31 and 1.36. In general, K = 1.34 is adopted), a difference between the arterial oxygen content and the mixed venous oxygen content, that is, the value of (Ca02-Cv02) can be represented by the following formula (3).

$$(CaO2-CvO2) = K \times Hb \times (SaO2-SvO2) \qquad \text{Formula (3)}$$

In the formula (3), K represents an oxygen binding amount per unit hemoglobin (1 g of hemoglobin), Hb represents the amount of hemoglobin in blood, Sa02 represents an arterial oxygen saturation, and Sv02 represents a mixed venous oxygen saturation. Here, Sa02 and Sv02 are each a fractional oxygen saturation. Abnormal hemoglobin (carboxyhemoglobin or methemoglobin) is hemoglobin which cannot bind to oxygen. In the case where the ratio of abnormal hemoglobin is large, when the functional oxygen saturation (the amount of oxygenated hemoglobin to the sum of the amount of oxygenated hemoglobin and the amount of deoxygenated hemoglobin) is substituted in the formula (3), CaO2 or CvO2, which is a blood oxygen content, becomes a value having an error. Due to this, in the case where the amount of abnormal hemoglobin is small, the functional oxygen saturation may be used in the formula (3), however, in the case where the amount of abnormal hemoglobin is large, it is not preferred to use the functional oxygen saturation in the formula (3).

Therefore, the mixed venous oxygen saturation can be calculated based on the formula (4) modified from the above formula (2) and formula (3). That is, the mixed venous oxygen saturation can be estimated from the calculation formula (formula (4)) based on the Fick's formula (the law of blood circulation) and the oxygen binding amount per unit hemoglobin (for example, 1 g of hemoglobin).

$$SvO2 = SaO2 - (VO2 / (K \times Hb \times CO)) \qquad \text{Formula (4)}$$

In the formula (4), SvO2 represents a mixed venous oxygen saturation, Sa02 represents an arterial oxygen saturation, V02 represents an oxygen consumption, Hb represents the amount of hemoglobin in blood, CO represents a cardiac output, and K represents an oxygen binding amount per unit hemoglobin (for example, 1 g).

The Sv02 estimation section 15 calculates an estimation value of the mixed venous oxygen saturation by substituting the arterial oxygen saturation, the amount of hemoglobin in blood, the cardiac output, and the oxygen consumption calculated by the respective calculation sections (the blood index calculation section 12, the cardiac output calculation section 13, and the oxygen consumption calculation section 14) in the formula (4). The Sv02 estimation section 15 inputs the calculated estimation value of the mixed venous oxygen saturation to the output section 16.

The output section 16 outputs various biological waveforms or the measurement values of various vital signs of a test subject. Here, the "output" may be a display output on a display or may be an output on a paper by printing.

Further, the "output" is a concept including an output of an alarm sound when any of the vital signs is abnormal. Therefore, the output section 16 is a display (and a peripheral circuit of the display), a printer (and a peripheral circuit thereof), a speaker (and a peripheral circuit thereof), or the like provided for the biological information measurement device 1. The output section 16 outputs the mixed venous oxygen saturation estimated by the Sv02 estimation section 15. For example, the output section 16 displays a measurement value or a trend graph of the mixed venous oxygen saturation on a display. The output section 16 may also display a measurement value or a measurement waveform of a general vital sign (a blood pressure or a body temperature) on the display in addition thereto.

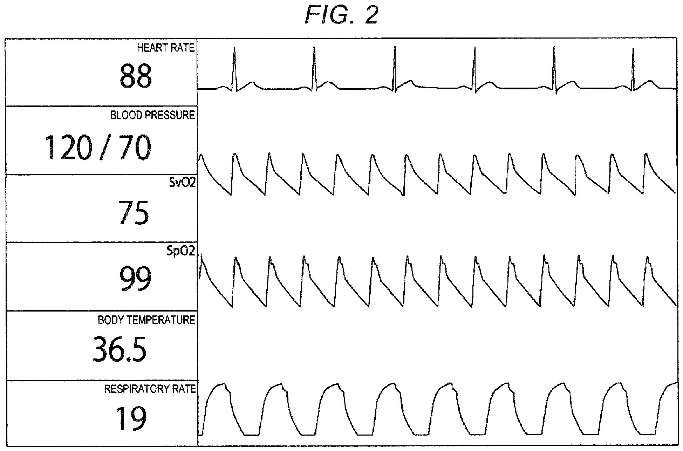

Fig. 2 is a view showing one example of a display screen on which the output section 16 performs displaying. As shown in the drawing, on the display screen, an estimated value of the mixed venous oxygen saturation (SvO2) is displayed along with a heart rate and a blood pressure. The display screen shown in Fig. 2 is merely an example, and the estimated value of the mixed venous oxygen saturation may not only be displayed by a numerical value, but also may be displayed by a trend graph (a form by which a change over time is found).

Next, an effect of the biological information measurement device 1 according to this embodiment will be described. As described above, it is known that a blood index (an arterial oxygen saturation or the amount of hemoglobin in blood) associated with oxygen transport, an oxygen consumption, and a cardiac output can be noninvasively and accurately acquired by the function of a biological information monitor used in a general hospital. The Sv02 estimation section 15 estimates a mixed venous oxygen saturation by substituting these parameters in a calculation formula derived from the blood circulation and the oxygen transport amount per unit hemoglobin. The SvO2 estimation section 15 uses parameters which can be noninvasively and accurately calculated in the calculation, and therefore, the mixed venous oxygen saturation can be continuously and accurately estimated.

The techniques described in the above-mentioned Patent Documents 1 to 3 each require an exclusive sensor (in the technique described in patent Document 1, a light-emitting element for deep blood vessels, etc., in the technique described in patent Document 2 , a photoacoustic imaging sensor, and in the technique described in patent Document 3, an NIRS sensor). Due to this, these techniques have problems that the configuration of the device is complicated, and also the cost is high. Further, it is necessary to apply light to a blood vessel to be measured from immediately above the vessel, and therefore, more accurate measurement may not be able to be performed due to a lack of experience of a medical worker or the like.

On the other hand, the biological information measurement device 1 according to this embodiment can estimate the mixed venous oxygen saturation (Sv02) by the configuration of a biological information monitor using a common probe 20 for multiwavelength measurement, an electrocardiographic electrode 30, and a respiration sensor 40. That is, the mixed venous oxygen saturation can be estimated while avoiding the complication of the device or the increase in cost due to the adoption of an exclusive sensor. Further, the above-mentioned parameters can be noninvasively and accurately measured regardless of the experience or skill of a medical worker. Therefore, even if any medical worker is in charge of the test subject, the mixed venous oxygen saturation can be accurately estimated.

The transmitted light or the reflected light received by the probe 20 can also be used for the calculation of the amount of hemoglobin in blood along with the calculation of the arterial oxygen saturation. The transmitted light or the reflected light can be used in common for the calculation of a plurality of parameters, and therefore, the device can be simplified.

It is preferred that the blood index calculation section 12 calculates a value obtained by subtracting the amount of abnormal hemoglobin from the total amount of hemoglobin in blood (in other words, only the amount of normal hemoglobin) as the amount of hemoglobin in blood. According to this, the effect of abnormal hemoglobin which cannot transport oxygen can be cancelled, and thus, the mixed venous oxygen saturation can be more accurately estimated.

Hereinabove, the invention made by the present inventors has been specifically described based on embodiments, however, the invention is not limited to the above-mentioned embodiments, and it is needless to say that various modifications can be made without departing from the gist of the invention.

The calculation methods of the respective parameters described above are merely examples, and other methods may be used. That is, the Sv02 estimation section 15 may estimate the mixed venous oxygen saturation by substituting the measurement values of the respective parameters obtained by an arbitrary method which is noninvasive and is not affected by the experience of a medical worker in the above-mentioned calculation formula.

For example, the amount of hemoglobin in blood may be measured from blood collected in advance from a test subject. In this case, it is only necessary to adopt a configuration in which the probe 20 or the blood index calculation section 12 calculates only the arterial oxygen saturation (that is, a general configuration in which the measurement of an arterial oxygen saturation is performed).

Further, the cardiac output calculation section 13 may calculate the cardiac output from a blood flow signal as described in Patent Document 8 (JP-A-2003-220045) or Non-Patent Document 5 (Internet <URL: http://www.kykb.jp/manatecl.html> searched on February 13, 2016). Further, the cardiac output calculation section 13 may calculate the cardiac output noninvasively based on the value of a blood pressure measured from a finger or the like (Patent Document 9 (JP-T-2002-541961) or Non-Patent Document 6 (Internet <URL: http://www.edwards.com/jp/pro-

fessionals/products/hemodynamic_monitoring/co_sv/clearsight/#> searched on February 13, 2016)). The cardiac output calculation section 13 may also calculate the cardiac output noninvasively using a method called a so-called "reactance method" (Patent Document 10 (JP-T-2014-521433) or Non-Patent Document 7 (Internet <URL: http://www.imimed.co.jp/product/monitor/detail/starling_sv .html> searched on February 13, 2016)). Further, as described in Patent Document 11 (JP-A-2006-231012), an oxygen circulation time is measured, and an estimation value of the cardiac output correlated with the oxygen circulation time may be calculated using a regression equation.

That is, the cardiac output calculation section 13 may calculate the cardiac output based on a biological signal acquired from a test subject using a noninvasive method.

At least part of the processing performed by the above-mentioned various processing sections (the light emission control section 11, the blood index calculation section 12, the cardiac output calculation section 13, the oxygen consumption calculation section 14, and the Sv02 estimation section 15) may be realized by causing a CPU (Central Processing Unit, not shown in Fig. 1) provided in the biological information measurement device 1 to execute a program.

Here, the program is stored using various types of non-transitory computer readable media and can be supplied to a computer. The non-transitory computer readable media include various types of tangible storage media. Examples of the non-transitory computer readable media include magnetic recording media (such as flexible disks, magnetic tapes, and hard disk drives), magneto-optical recording media (such as magneto-optical disks), CD-ROM (Read Only Memory), CD-R, CD-R/W, semiconductor memories (such as mask ROM, PROM (Programmable ROM), EPROM (Erasable PROM), flash ROM, and RAM (random access memory). Further, the program may be supplied to a computer through any of various types of transitory computer readable media. Examples of the transitory computer readable media include electrical signals, optical signals, and electromagnetic waves. The transitory computer readable media can supply the program to a computer through a wired communication channel such as an electrical wire or an optical fiber or a wireless communication channel.

**Claims**

1. A biological information measurement device (1), comprising:

   a blood index calculation section (12) which calculates at least part of a blood index associated with oxygen transport based on transmitted light or reflected light when an arbitrary body part of a test subject is irradiated with light with a plurality of wavelengths;
   an oxygen consumption calculation section (14) which calculates an oxygen consumption based on a gas concentration in inspired air, a gas concentration in expired air, and a ventilation amount of the test subject;
   a cardiac output calculation section (13) which calculates a cardiac output based on a biological signal acquired by a noninvasive method from the test subject; and
   an SvO2 estimation section (15) which estimates the mixed venous oxygen saturation of the test subject by substituting the blood index, the oxygen consumption, and the cardiac output in a calculation formula derived from the law of blood circulation and an oxygen binding amount per unit hemoglobin.

2. The biological information measurement device (1) according to claim 1, wherein the calculation formula is represented by the following formula:

$$SvO2 = SaO2 - (VO2 / (K \times Hb \times CO)) \qquad Formula\ (4)$$

   wherein Sv02 represents a mixed venous oxygen saturation, Sa02 represents an arterial oxygen saturation, V02 represents an oxygen consumption, Hb represents the amount of hemoglobin in blood, CO represents a cardiac output, and K represents an oxygen binding amount per unit hemoglobin (for example, 1 g) .

3. The biological information measurement device (1) according to claim 2,
   wherein the K is a value of 1.3 to 1.4.

4. The biological information measurement device (1) according to any one of claims 1 to 3,
   wherein the light with a plurality of wavelengths includes light which is absorbed by water, and
   the blood index calculation section calculates the amount of hemoglobin in blood along with an arterial oxygen saturation based on the transmitted light or the reflected light.

5. The biological information measurement device (1) according to any one of claims 1 to 4,
wherein the blood index calculation section calculates a value obtained by subtracting the amount of abnormal hemoglobin from the total amount of hemoglobin in the blood of the test subject as the amount of hemoglobin in blood constituting the blood index.

6. A mixed venous oxygen saturation estimation method, comprising:

a blood index calculation step of calculating at least part of a blood index associated with oxygen transport based on transmitted light or reflected light when an arbitrary body part of a test subject is irradiated with light with a plurality of wavelengths;
an oxygen consumption calculation step of calculating an oxygen consumption based on a gas concentration in inspired air, a gas concentration in expired air, and a ventilation amount of the test subject;
a cardiac output calculation step of calculating a cardiac output based on a biological signal acquired by a noninvasive method from the test subject; and
an Sv02 estimation step of estimating the mixed venous oxygen saturation of the test subject by substituting the blood index, the oxygen consumption, and the cardiac output in a calculation formula derived from the law of blood circulation and an oxygen binding amount per unit hemoglobin.

7. A program which causes a computer to execute
a blood index calculation step of calculating at least part of a blood index associated with oxygen transport based on transmitted light or reflected light when an arbitrary body part of a test subject is irradiated with light with a plurality of wavelengths,
an oxygen consumption calculation step of calculating an oxygen consumption based on a gas concentration in inspired air, a gas concentration in expired air, and a ventilation amount of the test subject,
a cardiac output calculation step of calculating a cardiac output based on a biological signal acquired by a noninvasive method from the test subject, and
an Sv02 estimation step of estimating the mixed venous oxygen saturation of the test subject by substituting the blood index, the oxygen consumption, and the cardiac output in a calculation formula derived from the law of blood circulation and an oxygen binding amount per unit hemoglobin.

# FIG. 1

FIG. 2

| HEART RATE | 88 |
| BLOOD PRESSURE | 120 / 70 |
| SvO2 | 75 |
| SpO2 | 99 |
| BODY TEMPERATURE | 36.5 |
| RESPIRATORY RATE | 19 |

Europäisches
Patentamt

European
Patent Office

Office européen
des brevets

# EUROPEAN SEARCH REPORT

**Application Number**

EP 17 15 1757

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DAVIES C ET AL: "APPLICATION OF THE KUBELKA MUNK EQUATION TO CHARACTERIZING A REFLECTANCE PULSE OXIMETER", IMAGES OF THE TWENTY FIRST CENTURY. SEATTLE, NOV. 9 - 12, 1989; [PROCEEDINGS OF THE ANNUAL INTERNATIONAL CONFERENCE OF THE ENGINEERING IN MEDICINE AND BIOLOGY SOCIETY], NEW YORK, IEEE, US, vol. 11 PART 04/06, 9 November 1989 (1989-11-09), pages 1095-1097, XP000129284, * page 1095 - page 1097 * | 1-7 | INV. A61B5/1455 A61B5/083 ADD. A61B5/029 A61B5/00 A61B5/08 A61B5/091 |
| A | US 2005/139213 A1 (BLIKE GEORGE T [US]) 30 June 2005 (2005-06-30) * the whole document * | 1-7 | |
| A | US 6 743 172 B1 (BLIKE GEORGE T [US]) 1 June 2004 (2004-06-01) * the whole document * | 1-7 | |
| A | US 5 634 461 A (FAITHFULL NICHOLAS S [US] ET AL) 3 June 1997 (1997-06-03) * the whole document * | 1-7 | **TECHNICAL FIELDS SEARCHED (IPC)** <br> A61B |

The present search report has been drawn up for all claims

| Place of search | Date of completion of the search | Examiner |
|---|---|---|
| The Hague | 20 July 2017 | Faymann, Juan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 213 686 A1**

## ANNEX TO THE EUROPEAN SEARCH REPORT
## ON EUROPEAN PATENT APPLICATION NO.

EP 17 15 1757

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2017

| Patent document cited in search report | | | Publication date | Patent family member(s) | | | Publication date |
|---|---|---|---|---|---|---|---|
| US 2005139213 | A1 | | 30-06-2005 | NONE | | | |
| US 6743172 | B1 | | 01-06-2004 | NONE | | | |
| US 5634461 | A | | 03-06-1997 | AU | 717271 | B2 | 23-03-2000 |
| | | | | CA | 2221995 | A1 | 19-12-1996 |
| | | | | CN | 1192130 | A | 02-09-1998 |
| | | | | EP | 0830078 | A1 | 25-03-1998 |
| | | | | HU | 9802264 | A2 | 01-02-1999 |
| | | | | JP | H11507276 | A | 29-06-1999 |
| | | | | NO | 975319 | A | 09-01-1998 |
| | | | | NZ | 311397 | A | 28-01-2000 |
| | | | | PL | 323873 | A1 | 27-04-1998 |
| | | | | US | 5634461 | A | 03-06-1997 |
| | | | | WO | 9639928 | A1 | 19-12-1996 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010524598 T
- WO 13112812 A
- US 20120065485 A
- JP 8322822 A
- JP 2005312947 A
- JP 2002228579 A

- JP 2002315739 A
- JP 2003220045 A
- JP 2002541961 T
- JP 2014521433 T
- JP 2006231012 A

**Non-patent literature cited in the description**

- **TAKUO AOYAGI.** Pulse Oximeter no Tanjo to Riron. *The Journal of Japan Society for Clinical Anesthesia,* 1990, vol. 10 (1), 1-11